(19) Europäisches Patentamt
European Patent Office
Office européen des brevets

(11) **EP 4 157 431 B1**

(12) **EUROPEAN PATENT SPECIFICATION**

(45) Date of publication and mention
of the grant of the patent:
**24.04.2024   Bulletin 2024/17**

(21) Application number: **21742484.5**

(22) Date of filing: **24.05.2021**

(51) International Patent Classification (IPC):
**A61N 1/04** *(2006.01)*     **A61N 1/36** *(2006.01)*
**A61N 1/32** *(2006.01)*     A61N 1/20 *(2006.01)*
A61F 13/00 *(2024.01)*     A61F 13/02 *(2024.01)*

(52) Cooperative Patent Classification (CPC):
**A61N 1/0468; A61N 1/0492; A61N 1/326;**
**A61N 1/36034;** A61F 13/0206; A61F 13/05;
A61N 1/205

(86) International application number:
**PCT/IB2021/054491**

(87) International publication number:
**WO 2021/240344 (02.12.2021 Gazette 2021/48)**

(54) **LAYERED ELECTRO-STIMULATING DRESSING**

GESCHICHTETER ELEKTROSTIMULIERENDER VERBAND

PANSEMENT ÉLECTRO-STIMULANT EN COUCHES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.05.2020   PL 43406820**

(43) Date of publication of application:
**05.04.2023   Bulletin 2023/14**

(73) Proprietor: **PREVLLY SPOLKA Z OGRANICZONA**
**ODPOWIEDZIALNOSCIA**
**20-883 Lublin (PL)**

(72) Inventors:
• **ZGIERSKI, Jeremi**
**20-539 Lublin (PL)**
• **MAZUR, Rafal**
**26-600 Radom (PL)**
• **TORUN, Mateusz**
**21-065 Rybczewice (PL)**

(74) Representative: **Bury & Bury**
**Ul. Przyczolkowa 124**
**02-968 Warszawa (PL)**

(56) References cited:
**US-A1- 2005 085 751     US-A1- 2008 103 462**
**US-A1- 2015 257 970     US-A1- 2018 200 514**
**US-A1- 2019 091 466**

## Description

Invention concerns a dressing, in particular for treatment and for prevention of pressure ulcers and a method of generating signals for electrostimulation by means of this dressing.

A prolonged tissue compression, occurring while lying in bed during chronic diseases, results in an ischaemia of tissues located between bone structures and a ground and as a consequence in a formation of wounds - so called pressure ulcers.

The pressure ulcers extend the time of stay of patients in hospital and they significantly increase costs of hospitalization, they complicate the process of treatment of a basic disease, they require an increased engagement of medical personnel and they can be a cause of a sepsis - a human life-threatening condition.

In the state of the art, various techniques of preventing the pressure ulcers are known, including mechanical installations mounted in the bed itself, e.g. a device according to patent PAT.192860, overlays for mattresses as disclosed e.g. in patent PAT.213182, or sole mattresses as disclosed e.g. in patent PAT.234659 or PAT.219452. Dressings as disclosed in e.g. a translation of European patent EP2568939T3 are also used. In Japanese patent application number JP2005021263A, a gel material is disclosed, suitable for using in dressings, keeping shape and distributing/spreading pressure. Methods for stimulating work of muscles by enforcing the position change by electrostimulation are also known - for example from a an American patent application number US20030050675. From the publication of international patent application number WO200771642, a dressing with the electrodes for electrostimulation is also known. Publication no US2015257970A1 of US patent application discloses a layered electro-stimulating dressing comprising a substrate, an absorption layer and an electro-stimulating layer comprising a first electrode and a second electrode. Electrostimulation is also discussed in US 2018200514A1, US2019091466A1, US2008103462A1 and US2005085751A1.

Those solutions are not effective enough, they are too complicated, they require the use of bulky equipment, relatively excessively annoying for a patient. It concerns particularly the electrostimulation, in which it is difficult to apply electrodes and it is difficult to provide stability of operating conditions - particularly skin resistance which is changing with humidity of the patient's skin with wounds. As a result, electrostimulation of the patients at risk of the pressure ulcers often requires a regular supervision and regulation, preferably by a qualified person.

The objective of the invention is to provide a solution for the problem of the effective prevention of the pressure ulcers without a need for the regular participation of the qualified person.

The present invention provides a layered electro-stimulating dressing according to claim 1.

The dependent claims define further features and embodiments of the present invention.

The layered electro-stimulating dressing comprises a substrate, an absorption layer, an electro-stimulating layer comprising a first electrode and a second electrode and a generation system for generation of stimulating signals, wherein the first electrode and the second electrode are connected to the generation system of stimulating signals, wherein the generation system for generation of stimulation signal comprises a control system, a power supply system connected to it and a generator. The substrate constitutes a pressure distribution layer made of a gel having hyperplastic properties or made of thermoplastic elastomer formed into honeycomb structure, wherein the generator is adapted for generating stimulating signals comprising impulses with an electric current falling within a range of 4 mA to 100 mA and a duration falling within a range of 50 $\mu$s to 2.5 ms, with a repetition rate falling within a range of 10 Hz to 60 Hz wherein the impulses are repeated for a stimulation time lasting from 3 to 7 seconds at intervals with a length of two to 10 times longer than the stimulation time. Electric currents lower than 60 mA are barely perceptible even for patients with low pain threshold. The impulses with the parameters falling within this range allow for effective stimulation and are tolerable for the patient. At the same time, the use of the pressure distribution layer constitutes a physical mechanism preventing degeneration of tissues. The absorption layer absorbs discharges and limits a risk of infections. As a result, an effectiveness of the dressing exceeded initial expectations.

The dressing advantageously further comprises a voltage sensor between the first electrode and the second electrode. The voltage sensor facilitates the control of the operation of generation system.

The dressing advantageously further comprises a sensor of current flowing via the electrodes. In combination with the voltage sensor it allows for measurement of the impedance of the tissues.

The dressing advantageously comprises a layer, in which at least one sensor is placed, chosen from a group comprising a pressure sensor and a temperature sensor connected to the control system. Using of the sensors in the dressing allows for recording the work conditions. A correlation of recording with a state of the patient allows for a therapy adaptation.

The dressing advantageously has an outer insulation layer. This layer prevents damages.

The generation system of stimulating signals is advantageously integrated with the dressing layers. Pressure distribution layer is particularly applicable for that purpose due to its mechanical properties giving opportunity to make generation system imperceptible.

Alternatively, the generation system of stimulating signals is housed in a separate housing and is adapted for connection to the electrodes by means of elastic electrical wires.

The control system is advantageously provided with a radio communication system. Thanks to this it is possible to control the operation of the system and to read and register the parameters from the sensors by means of the external device. It facilitates diagnostics and the analysis of the therapy effectiveness.

The dressing advantageously has at least one layer comprising hydrocolloid substance. Such solution allows for using the dressing for providing active substances to the skin of the patient in a long release process. Thanks to this it is possible to additionally achieve synergy with the electrostimulation. The hydrocolloids provide also a possibility of improving absorption properties.

The dressing advantageously comprises at least one layer comprising a gel. Such layers contribute to better pressure distribution.

The generator is advantageously adapted for generating stimulating signals comprising impulses, repeated with the frequency falling within a range of 10 to 60 Hz, the duration falling within a range of 100 $\mu$s to 600 $\mu$s, and in particular falling within a range of 200 $\mu$s do 350 us. Such impulses are less severe for the patient and they provide electrostimulation. As a result, better effects are achieved in a view of improving motivation of the patient.

A method of generating signals for electrostimulation performed automatically by means of a dressing according to the invention is defined by claim 13.

Advantageously, the impulse duration is falling within a range of 100 $\mu$s to 600 $\mu$s, in particular of 200 $\mu$s to 350 $\mu$s.

The invention is defined by the appended claims and is explained below in details with reference to the attached drawings in which Fig. 1a presents schematically a cross-section of the dressing according to an embodiment of the invention, Fig. 1b presents schematically a cross section of another embodiment of the invention, Fig. 2 presents a schematic diagram of the connections of the electrical elements of the dressing, Fig. 3a-e present example embodiments of the dressing in a top view, Fig. 4 presents an alternative example embodiment of the dressing in a perspective view of partial cross-section, Fig 5 a and b illustrate waveforms of the stimulating signals with impulses, whereas Fig. 6 presents stimulation with indication of stimulation time $t_1$ and intervals $t_2$.

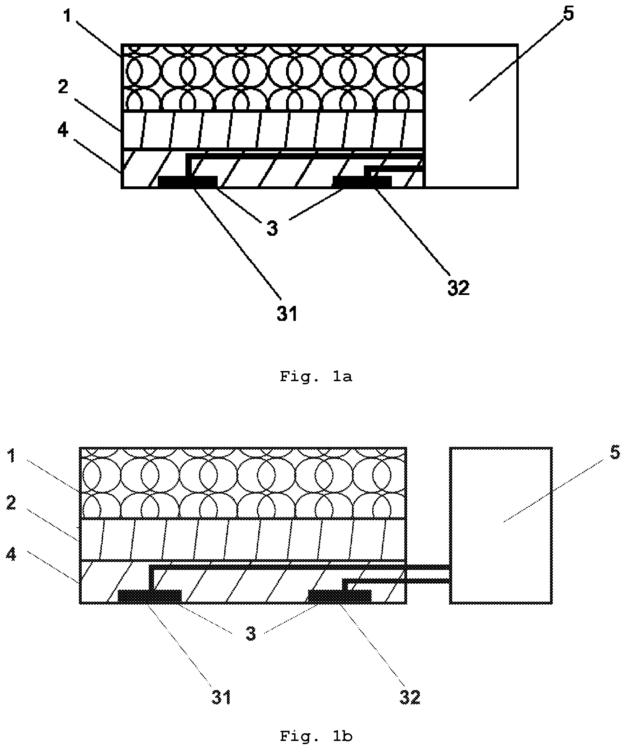

An example of the layered electro-stimulating dressing according to the invention is presented schematically in Fig. 1a. The outer layer 1 constitutes a pressure distribution layer made of a gel material with hyperelastic properties. A term of hyperelasticity is known in the art and it constitutes a combination of the conditions concerning hardness and elastic deformation capacity. The most suitable materials for pressure distributions were satisfying both of the following conditions: having hardness less or equal to 40° Sh A and being able to linear extension of 300% or more. Other synthetic and organic materials capable of spreading/distributing pressure due their structure or shape/geometry are also applicable and can be selected in relatively simple experiments, in which a sheet of material under test is located on pressure sensing mate to compare pressure distribution of predefined object with respect to reference material. The pressure distribution layer not only reduces the risk of pressure ulcers formation by distributing the pressure on the larger area, but also enables more stable electric contact of the electrodes with the skin. In general, good effects were obtained by using the pressure distribution layers made of thermoplastic elastomers TPE formed into a spatial structure - in particular "honeycomb" type.

Under the pressure distribution layer there is an absorption layer 2 made of soft absorptive material keeping discharges. Optionally, this layer is soaked in germicides and/or it is connected to the hydrocolloid layer adapted for receiving active substance and for its progressive releasing. A separate hydrocolloid layer (not shown in figures) may also be provided. The absorption layer absorbs sweat and other discharges and not only decreases the risk of skin maceration but also provides reduction of electrochemical conditions changes of a contact of the electrode/skin of the patient.

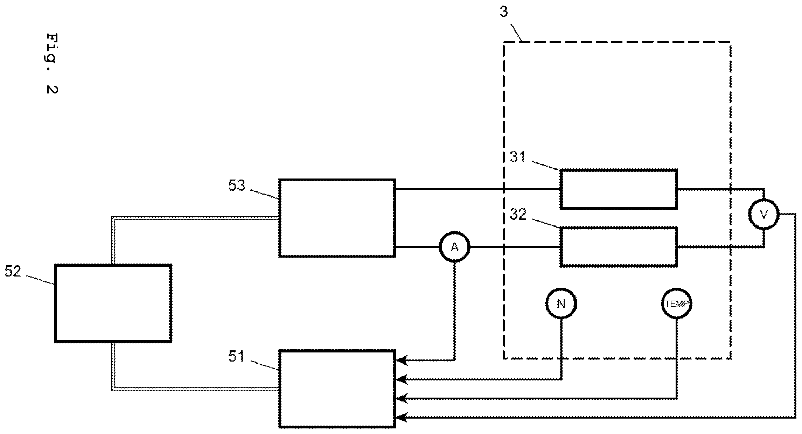

The electro-stimulating layer 3 is located on the same level as the adhesive layer 4 or optionally can be provided below it. The electro-stimulating layer 3 comprises the first electrode 31 and the second electrode 32, although it is possible to use more such electrodes. The first electrode 31 and the second electrode 32 are connected to the generation system 5 of stimulating signals provided with the control system 51, the power supply system 52 connected to it and the generator 53. The schematic diagram of connections is shown in Fig. 2.

As the control system 51, the microprocessor from STM32Lxx family was used. Alternatively also 32-bit ARM Cortex-M can be applied or whole microcontroller system STM32G071.

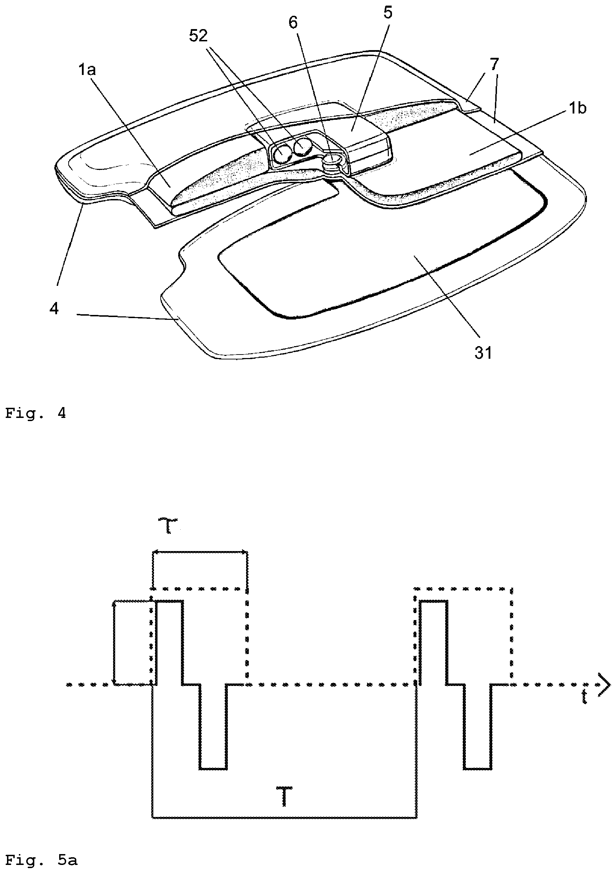

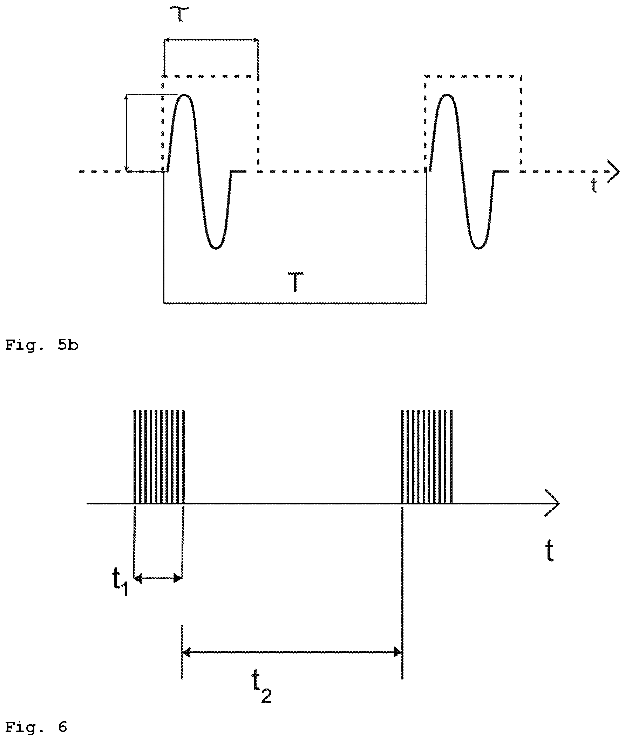

The generator 53 is adapted for generating stimulating signals with the electric current falling within a range of 4 mA to 100 mA and duration falling within a range of 50 $\mu$s to 2.5 ms, with repetition rate falling within a range of 10 to 60 Hz. Better effects were obtained by using the impulses with duration falling within a range of 100 $\mu$s to 600 us. An exemplary waveform of an excitation signal is shown in Fig. 5a with an indication of the period on the basis of which the pulse repetition rate is determined - for example 40 Hz. In Fig. 5b, a single impulse with an indication of an amplitude of current I is shown - e.g. 10 mA and impulse duration - e.g. 300 $\mu$s.

In the simplest embodiment an energy storage can be used as the power supply system 52 - a battery or a rechargeable accumulator. Due to relatively short time of active operating time of the device, the energy storage can be supported by an energy harvesting module (energy harvesting), not shown in the figures. The energy harvesting modules known in the state of the art allow for energy generation by use of the difference between the temperature of body and the ambient temperature or inertial energy associated with patient movements. In some configurations it may also be necessary to apply converters providing desirable voltage.

The power supply system 52 can also constitutes a power supply unit connected to a power grid.

Better control of the therapy can be obtained by using additional sensors connected to the control system 51: voltage sensor V between the first electrode 31 and the second electrode 32, sensor of current A flowing via the electrodes, a pressure sensor N and a temperature sensor TEMP. Such sensors allow for obtaining information on exposures to which the tissues are exposed and on their condition. As the control system 51 a digital system can be used, launching various programmes depending on the settings of the sensors.

Advantageously, the dressing is equipped with a radio communication module - in particular Blueooth Low Energy or NFC. Such module allow programming the control system by means of typical portable devices as well as reading from it indications of the sensors by means of the portable devices.

The dressing is also worth equipping with an outer insulating layer 7. The pressure distribution layer is covered by it. A range of materials can be used, in particular medical silicone or film/foil.

One layer can perform more than one function. For example, the absorption layer can also support functions of the pressure distribution layer - by discharge gelling - in order to obtain a gel having a cushioning effect.

The layers can be of various shapes. For example the electro-stimulating layer can be placed only in the area corresponding to part of the patient's body which is a subject to the electro-stimulation - it is cut by mapping the shape of the muscle. In the remaining part of the dressing, such layer can be absent or it can be on the whole surface of the dressing. The electro-stimulating layer can be of the shape of single foci with the electrodes and those foci can be surrounded by the absorption layer or it can cover the majority of the dressing and it may have perforations, by which the absorption layer may be in contact with skin. The absorption layer can be in the shape of cylinders or other geometrical figures. The elements of the absorption layer can be connected to each other at higher level, further away from the skin. The pressure distribution layer may constitute an isolated layer located above other layers or may penetrate through other layers via perforations made for it.

According to the invention, the generation system 5 with the control system 51 and the supply system 52 can be provided in the dressing itself, permanently attached to the layers or brought into a separate housing and connected to the electrodes by wires, as shown in Fig. 1b. This second solution has such advantage, that it distributes pressure more easily, and a connection plug - socket can be provided, enabling a connection of various generation systems 5. As a result, simple non-regulated systems can be used and still there is a possibility to match the insensitivity of stimulation to the patient's condition.

The generation system 5 of stimulating signals can be integrated in the layers of the dressing and it can have connection to the electrodes passing through the structure. The generation system of stimulating signals is located between the layers or next to them on the edge of the dressing - as shown in Fig. 1b. The most advantageous, so that then the generation system is adjustable.

The full integration of the generation system 5 of stimulating signals has a range of advantages, in particular ease and simplicity of use and application, what is limited to sticking of the dressing in the right place. The risk of loosening, disconnection, or tearing of cables due to patient movements is also avoided. However, such solution requires the use of soft and elastic electronic components, which as a rule have lower parameters. Additional disadvantage is complication of charging or changing the battery, and as a consequence, a disposability of the overall product.

Alternatively, the generation system 5 of stimulating signals can have a separate housing and can be connected to the electrodes 31, 32 by elastic electronic connections going beyond the structure of the layers.

Providing the generation system 5 of signal beyond the structure of the layers and connection by conductive tapes with the electrodes, allows for convenient positioning of the generation system 5 of impulses in respect of the structure of the layers of the dressing, e.g. by laying it down freely on a bed, in a pocket of a bed, or for fixing it to the opposite part of the body to the area affected to pressure ulcers. However, such solution requires greater commitment of the person using/operating and increases the risk of interruption of electrical contact. However, in such configuration, components comprising hard elements can be used - in particular the energy storage with higher capacity, without the need of additional means for distribution of the pressure of those elements. Such solution also allows for the possibility of the use of one generation system of the stimulating impulses, several times during the exchange of the dressings. It is also easier to provide the possibility of recharging several times and thus prolonging the lifetime and avoiding the need to use the whole device once.

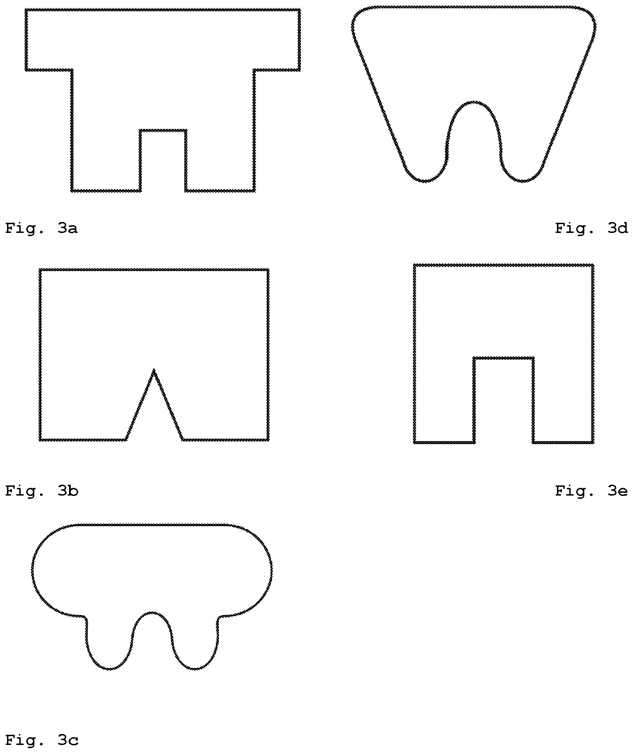

The shape of the dressing according to the invention can be chosen to the area subjected to the treatment or prevention. In Fig. 3a-e the embodiments of the dressing are shown in the top view, adapted for prevention of the pressure ulcers in the neighborhood of a sacrum.

The embodiment shown in Fig. 3a has two side wings: one extends leftward, one extends rightward and two wings are extended downwards. The advantage of that shape is a large skin contact surface, below which a muscle is located. Side wings protect the patient during body rotation, an indentation between the lower wings is an indentation for an intergluteal cleft, what allows for nursing of the patient without the need of removal of the dressing in a case of uncontrolled defecation. Some disadvantage of such shape is a large number of corners, which increases a risk of uncontrolled removal.

In the embodiment shown in Fig 3b, the surface of adhesion to the skin is even greater. Two wings extended downwards are provided, put on the gluteals, and the indentation between them for the intergluteal cleft. This allows for nursing the patient without a need of removal of the dressing in a case of uncontrolled defecation. Thus, the greater width of the dressing was provided while limiting the range on the gluteals. The lack of the wings increases a risk in rotary movements. A sharp indentation between the wings enforces a smaller distance from the intergluteal cleft, what increases a risk of transmission of pollutants under the dressing. The simplicity of the shape makes it easy to manufacture.

In the Fig. 3c, the embodiment with rounded corners and the side wings protecting the patient during body rotation is shown, indentation between the lower wings is an indentation for an intergluteal cleft, what allows for nursing of the patient without a need of removal of the dressing in a case of uncontrolled defecation. Rounded shapes affect advantageously adhesion security and reduce susceptibility to removal.

The embodiment shown in Fig. 3d has those advantages while providing increase of the surface of adhesion to the skin under which there is a muscle. Indentation between the lower wings of dressing is an indentation for the intergluteal cleft, what allows for nursing of the patient without a need of removal of the dressing in a case of uncontrolled defecation. Lower wings of the embodiment shown in Fig. 3d extend low down enabling placing the dressing in close proximity to a tuber ischiadicum when applying the dressing and in that location they provide benefit of distributing the pressure and supporting the muscle cushion. The connection of the low wings with the wings increases the surface and appears to offer less risk of removal due to the rounded shape of the dressing. The connection of the low wings with the wings can run as a straight line or as an arc (which will increase the surface of adhesion). The further advantage is high shape modifiability - the wings going downwards may be elongated, as well as they can be shortened. The same applies to expansion and contraction of the dressing.

Disadvantages of the greater surface of adhesion include the use of material during manufacturing and a need to provide an increased surface absorbing the discharges. Despite this, during tests, this shape gave the best results.

Simple, easy to make, scale and model embodiment is shown in Fig. 3e. It has the smallest surface of adhesion but sharp corners and a lack of the wings makes it susceptible to removal.

In all embodiments, the top edge of the dressing can be of the shape of an arc convex upward, what increases the surface and makes more spherical shape of the whole dressing, or in the standard shape of the straight line. Each shape can have straight, corrugated or notched edges, however straight or corrugated edges have performed the best.

An alternative embodiment of the dressing is shown in semi-foldout view, in perspective in Fig. 4. The adhesive layer 4 made of conductive silicone adheres directly to the skin, and above it there is the electro-stimulating layer 3 with the visible electrode 31. The pressure distribution layer was divided into an outer pressure distribution layer 1a and an internal pressure distribution layer 1b. In the outer pressure distribution layer 1a the generation system 5 of the stimulating signals is provided. As the supply system 52 the system of two batteries was used. The dressing was secured by the insulation layer 7. The layers are connected by welding and additionally by a clip 6. The disadvantage of such solution is that the generation system 5 of the stimulating signals and the energy storage are integrated in the dressing making pressure distribution more difficult. It is possible to use a single material to produce single layer that performs function of more than one layer. Examples of multifunction materials include hydrogels.

The housing of the generation system 5 of the stimulating signals is made of polymer PC - colourless, transparent, non-flammable and non-toxic with excellent mechanical strength. Alternatively, polymer ABS can be used, what makes the recycling easier but poses a slightly higher risk due to flammability. Polypropylene can also be used, which is perfectly physiologically inert and decomposes well but has low air permeability.

According to embodiment of the invention, the electrodes 31, 32 in the stimulating layer 3 are set up close to the outer boundaries of the dressing. In the dressings shown in Fig. 3a-e adapted to be located in the neighbourhood of a sacrum, the electrodes extend downwards wings of the dressing - thanks to this their position corresponds to the position of the muscles. In more complex embodiments, a much larger set of electrodes can be used and can be densified in the neighborhood of the muscles. This facilities effective carrying out of electrostimulation and this affects the choice of the dressing depending on the place of application.

The localization of the electrodes depends on the shape and the place of destination. This relationship is caused by various distribution of muscles and other tissues as well as their thickness in various parts of the body.

A method of generating signals for electrostimulation according to the invention is carried out most effectively by means of the dressing according to the invention. The method of generating signals for electrostimulation is realized automatically by means of the control system 51. The control system 51 is set so, that it generates a stimulating signal comprising impulses repeated during the stimulation with the frequency f=1/T - as shown in Fig. 5a. During stimulation time, the frequency of impulse repetition falls within a range of 10 Hz to 60 Hz. In the embodiment shown in Fig. 5a, this frequency is equal to f = 40 Hz and the impulses with the maximum current equal to I = 10 mA and pulse duration $\tau$ = 600 μs were used. Stimulation times falling in a range of 3 to 7 seconds were used. Patient's comfort generally requires the use of intervals in stimulation with the length of 2- to 10- times longer than stimulation time. The impulse shown in Fig. 5a is bipolar, almost rectangular but the negative part is separated from the positive part by the short zero section. Its occurrence limits the severity of the stimulation for the patient.

The impulse of an alternative shape is illustrated in Fig. 5b.

Particularly advantageous effects were observed with the use of the stimulation time $t_1$ falling within a range of 3 to 7 seconds and intervals $t_2$ being a multiple thereof:

$$\begin{cases} t_1 \in (3\ s;\ 7s) \\ t_2 \in (2t_1;\ 10t_1) \end{cases}$$

Establishing a training protocol requires research and observation. As a starting point, it is possible to take a scheme 4x per week, 1x or 2x per day, 30 minutes of the stimulation. Due to the muscle fatigue effect resulting from overtraining, a protocol of training requires adaptation. It can be done by a qualified person - e.g. a doctor or a physiotherapist. By providing an application for a smartphone communicating with the dressing, information about the schemes and their effects used by the qualified persons can by aggregated on the server. The dressing according to the invention ensures data collection and a selection of new schemes. Additionally it is possible to distribute better stress applied to skin by altering it by applying signals irrelevant for training but inducing single muscle contraction once in a longer time.

The effect of overtraining concerns in particular persons with severe muscle atrophy, in their case the training should be balanced and conservative.

Exceeding the current values indicated above can be painful or cause too long tetanus spasms, which are painful and it carries a risk of muscle rupture and damage to its structure, tendon rupture or even the detachment of a tendon attachment in the form of an avulsion fracture.

The further risk results from overtraining and muscles fatigue, due to too often training which does not allow for the regeneration of muscle tissue. In order to avoid it, in the structure of the dressing can be provided: the pressure sensors, temperature sensors, humidity sensors and communication modules. Such solution allow for transmission of readout data correlated with the stimulation. Used on external computing unit data can be used for training machine learning algorithms with data corresponding to large group of patients, to optimize parameters and stimulating signals.

It is crucial to stop the process of muscle atrophy. In a case of too often training without a sufficiently long break, a cumulation of microtrauma happens, consumption of glycogen reserves, resulting in muscle weakening and pain, which has a negative impact on training efficiency. On the other hand, too short exposition of the patient on the stimulating impulses results in the training is not effective - i.e. hypertrophy is not achieved or the progressive hypoplasia is not stopped.

The present invention is defined by the appended claims.

## Claims

1. A layered electro-stimulating dressing comprising a substrate, an absorption layer (2), an electro-stimulating layer (3) comprising a first electrode (31) and a second electrode (32) and a generation system (5) for generation of stimulating signals, wherein the first electrode (31) and the second electrode (32) are connected to the generation system (5) for generation of stimulating signals, wherein the generation system (5) for generation of stimulation signal comprises a control system (51), a power supply system (52) connected to it and a generator (53), wherein the substrate constitutes a pressure distribution layer (1) made of a gel having hyperplastic properties or made of thermoplastic elastomer formed into honeycomb structure, wherein the generator (53) is adapted for
generating stimulating signals comprising impulses with an electric current falling within a range of 4 mA to 100 mA and a duration falling within a range of 50 μs to 2.5 ms, with a repetition rate being within a range of 10 Hz to 60 Hz, wherein the impulses are repeated for a stimulation time lasting from 3 to 7 seconds at intervals with a length of two to 10 times longer than the stimulation time.

2. The dressing according to claim 1, **characterized in that** it further comprises a voltage sensor (V) between the first electrode (31) and the second electrode (32).

3. The dressing according to claim 1 or 2, **characterized in that** it further comprises an electric current sensor (A) of a current flowing via the electrodes (31, 32).

4. The dressing according to any of claims 1 to 3, **characterized in that** it comprises a layer, in which at least one sensor is placed, chosen from a group comprising a pressure sensor (N), a temperature sensor (TEMP) connected to the control system (51).

5. The dressing according to any of claims 1 to 4, **characterized in that** it has an outer insulation layer (7).

6. The dressing according to any of claims 1 to 5 **characterized in that** the generation system (5) of stimulating signals is integrated with the dressing layers.

7. The dressing according to any of claims 1 to 5, **characterized in that** the generation system (5) of stimulating signals is housed in a separate housing and is adapted for connection to the electrodes by means of elastic electrical wires.

8. The dressing according to any of claims 1 to 7, **characterized in that** the control system (51) is provided with a radio communication system.

9. The dressing according to any of claims 1 to 8, **characterized in that** it has at least on layer comprising hydrocolloid substance.

10. The dressing according to any of claims 1 to 9, **characterized in that** it comprises at least on layer comprising gel.

11. The dressing according to any of claims 1 to 10, **characterized in that** the generator (53) is adapted for generating stimulating signals comprising impulses with the duration ($\tau$) falling within the range of 100 $\mu$s to 600 $\mu$s, repeated with frequency being within the range of 10 to 60 Hz.

12. The dressing according to claim 11, **characterized in that** the generator (53) is adapted to generating stimulating signals comprising impulses with duration being within a range of 200 $\mu$s to 350 $\mu$s, repeated with the frequency falling within a range of 10 to 60 Hz.

13. A method of generating signals for electrostimulation performed automatically by means of a dressing as specified in any of claims 1 to 12, the generation system (5) of stimulating signals is set up so that it generates stimulating signals comprising impulses with duration ($\tau$) being within the range of 50 $\mu$s to 2.5 ms repeated with frequency falling within the range of 10 Hz to 60 Hz, repeated for a stimulation time ($t_1$) falling within a range of 3 to 7 seconds at intervals ($t_2$) with a length of 2 times to 10 times longer than the stimulation time ($t_1$).

14. The method according to claim 13, **characterized in that** the impulse duration ($\tau$) is within the range of 100 $\mu$s to 600 $\mu$s.

**Patentansprüche**

1. Geschichteter elektrostimulierender Verband umfassend ein Substrat, eine Absorptionsschicht (2), eine elektrostimulierende Schicht (3), die eine erste Elektrode (31) und eine zweite Elektrode (32) und ein Erzeugungssystem (5) zur Erzeugung von stimulierenden Signalen umfasst, wobei die erste Elektrode (31) und die zweite Elektrode (32) mit dem Erzeugungssystem (5) zur Erzeugung von stimulierenden Signalen verbunden sind, wobei das Erzeugungssystem (5) zur Erzeugung von stimulierenden Signalen ein Steuerungssystem (51), ein damit verbundenes Stromversorgungssystem (52) und einen Erzeuger (53) umfasst, wobei

das Substrat eine Druckverteilungsschicht (1) aus einem Gel mit hyperplastischen Eigenschaften oder aus einem thermoplastischen Elastomer, das zu einer Wabenstruktur geformt ist, bildet, wobei der Erzeuger (53) geeignet ist zum

Erzeugen von stimulierenden Signalen umfassend Impulse mit einem elektrischen Strom, der in einem Bereich von 4 mA bis 100 mA liegt, und einer Dauer, die in einen Bereich von 50 $\mu$s bis 2,5 ms fällt, wobei die Wiederholungsrate in einen Bereich von 10 Hz bis 60 Hz fällt, wobei die Impulse für eine 3 bis 7 Sekunden andauernde Stimulationszeit in Intervallen mit einer Länge, die zwei- bis 10-mal länger als die Stimulationszeit ist, wiederholt werden.

2. Verband nach Anspruch 1, **dadurch gekennzeichnet, dass** er ferner einen Spannungssensor (V) zwischen der ersten Elektrode (31) und der zweiten Elektrode (32) umfasst.

3. Verband nach Anspruch 1 oder 2, **dadurch gekennzeichnet, dass** er ferner einen elektrischen Stromsensor (A) für einen über die Elektroden (31, 32) fließenden Strom umfasst.

4. Verband nach einem der Ansprüche 1 bis 3, **dadurch gekennzeichnet, dass** er eine Schicht umfasst, in der mindestens ein Sensor angeordnet ist, der aus einer Gruppe umfassend einen Drucksensor (N), einen Temperatursensor (TEMP), die mit dem Kontrollsystem (51) verbunden sind, ausgewählt ist.

5. Verband nach einem der Ansprüche 1 bis 4, **dadurch gekennzeichnet, dass** er eine äußere Isolierschicht (7) aufweist.

6. Verband nach einem der Ansprüche 1 bis 5, **dadurch gekennzeichnet, dass** das Erzeugungssystem (5) von stimulierenden Signalen in die Verbandschichten integriert ist.

7. Verband nach einem der Ansprüche 1 bis 5, **dadurch gekennzeichnet, dass** das Erzeugungssystem (5) von stimulierenden Signalen in einem separaten Gehäuse untergebracht ist und für eine Verbindung mit den Elektroden mittels elastischer elektrischer Drähte geeignet ist.

**8.** Verband nach einem der Ansprüche 1 bis 7, **dadurch gekennzeichnet, dass** das Steuerungssystem (51) mit einem Funkkommunikationssystem ausgestattet ist.

**9.** Verband nach einem der Ansprüche 1 bis 8, **dadurch gekennzeichnet, dass** er mindestens eine Hydrokolloid-Substanz umfassende Schicht hat.

**10.** Verband nach einem der Ansprüche 1 bis 9, **dadurch gekennzeichnet, dass** er mindestens eine Gel umfassende Schicht hat.

**11.** Verband nach einem der Ansprüche 1 bis 10, **dadurch gekennzeichnet, dass** der Erzeuger (53) geeignet ist zum Erzeugen von stimulierenden Signalen, die Impulse mit einer Dauer (T), die in den Bereich von 100 $\mu$s bis 600 $\mu$s fällt, umfassen, die mit einer Frequenz im Bereich von 10 bis 60 Hz wiederholt werden.

**12.** Verband nach Anspruch 11, **dadurch gekennzeichnet, dass** der Erzeuger (53) geeignet ist zum Erzeugen von stimulierenden Signalen, die Impulse mit einer Dauer im Bereich von 200 $\mu$s bis 350 $\mu$s umfassen, die mit einer Frequenz, die in einen Bereich von 10 bis 60 Hz fällt, wiederholt werden.

**13.** Verfahren zum Erzeugen von Signalen für eine Elektrostimulation, das automatisch mittels eines Verbands nach einem der Ansprüche 1 bis 12 durchgeführt wird, wobei das Erzeugungssystem (5) von stimulierenden Signalen so eingerichtet ist, dass es stimulierende Signale erzeugt, die Impulse mit einer Dauer (1) im Bereich von 50 $\mu$s bis 2.5 ms, die mit einer Frequenz, die in den Bereich von 10 Hz bis 60 Hz fällt, wiederholt werden, für eine Stimulationszeit (ti), die in einen Bereich von 3 bis 7 Sekunden fällt, in Intervallen ($t_2$) mit einer Länge, die 2- bis 10-mal länger sind als die Stimulationszeit ($t_1$) ist, wiederholt werden.

**14.** Verfahren nach Anspruch 13, **dadurch gekennzeichnet, dass** die Impulsdauer (1) im Bereich von 100 $\mu$s bis 600 $\mu$s liegt.

**Revendications**

**1.** Pansement électro-stimulant en couches comprenant un substrat, une couche d'absorption (2), une couche électro-stimulante (3) comprenant une première électrode (31) et une deuxième électrode (32) et un système de génération (5) pour la génération de signaux de stimulation, dans lequel la première électrode (31) et la deuxième électrode (32) sont connectées au système de génération (5) pour la génération de signaux de stimulation, dans lequel le système de génération (5) pour la génération de signaux de stimulation comprend un système de commande (51), un système d'alimentation électrique (52) connecté à celui-ci et un générateur (53), dans lequel

le substrat constitue une couche de distribution de pression (1) constituée d'un gel ayant des propriétés hyperplastiques ou constituée d'un élastomère thermoplastique formé en une structure à nid d'abeilles, dans lequel le générateur (53) est adapté pour générer des signaux de stimulation comprenant des impulsions avec un courant électrique situé dans une plage de 4 mA à 100 mA et une durée située dans une plage de 50 $\mu$s à 2,5 ms, avec un taux de répétition situé dans la plage de 10 Hz à 60 Hz, dans lequel les impulsions sont répétées pour un temps de stimulation durant de 3 à 7 secondes à intervalles d'une longueur de deux à 10 fois plus longue que le temps de stimulation.

**2.** Pansement selon la revendication 1, **caractérisé en ce qu'**il comprend, en outre, un détecteur de tension (V) entre la première électrode (31) et la deuxième électrode (32).

**3.** Pansement selon la revendication 1 ou 2, **caractérisé en ce qu'**il comprend, en outre, un détecteur de courant électrique (A) d'un courant circulant via les électrodes (31, 32).

**4.** Pansement selon l'une quelconque des revendications 1 à 3, **caractérisé en ce qu'**il comprend une couche, dans laquelle est placé au moins un détecteur, choisi dans un groupe comprenant un détecteur de pression (N), un détecteur de température (TEMP) connecté au système de commande (51).

**5.** Pansement selon l'une quelconque des revendications 1 à 4, **caractérisé en ce qu'**il a une couche d'isolation externe (7) .

**6.** Pansement selon l'une quelconque des revendications 1 à 5, **caractérisé en ce que** le système de génération (5) des signaux stimulants est intégré aux couches du pansement.

**7.** Pansement selon l'une quelconque des revendications 1 à 5, **caractérisé en ce que** le système de génération (5) des signaux stimulants est logé dans un logement distinct et est adapté pour une connexion aux électrodes au moyen de fils électriques élastiques.

**8.** Pansement selon l'une quelconque des revendications 1 à 7, **caractérisé en ce que** le système de commande (51) est doté d'un système de radiocommunication.

**9.** Pansement selon l'une quelconque des revendications 1 à 8, **caractérisé en ce qu'**il a au moins une couche comprenant une substance hydrocolloïde.

**10.** Pansement selon l'une quelconque des revendications 1 à 9, **caractérisé en ce qu'**il comprend au moins une couche comprenant du gel.

**11.** Pansement selon l'une quelconque des revendications 1 à 10, **caractérisé en ce que** le générateur (53) est adapté pour générer des signaux stimulants comprenant des impulsions avec la durée ($\tau$) située dans la plage de 100 $\mu$s à 600 $\mu$s, répétée à une fréquence située dans la plage de 10 à 60 Hz.

**12.** Pansement selon la revendication 11, **caractérisé en ce que** le générateur (53) est adapté pour générer des signaux stimulants comprenant des impulsions ayant une durée située dans la plage de 200 $\mu$s à 350 ps, répétée à la fréquence située dans une plage de 10 à 60 Hz.

**13.** Procédé de génération de signaux pour une électrostimulation réalisée automatiquement au moyen d'un pansement comme spécifié dans l'une quelconque des revendications 1 à 12, le système de génération (5) de signaux stimulants est établi de manière à générer des signaux stimulants comprenant des impulsions ayant une durée ($\tau$) située dans la plage de 50 $\mu$s à 2,5 ms, répétée à une fréquence située dans la plage de 10 Hz à 60 Hz, répétée pour un temps de stimulation ($t_1$) situé dans une plage de 3 à 7 secondes à des intervalles ($t_2$) d'une longueur de 2 fois à 10 fois plus longue que le temps de stimulation ($t_1$).

**14.** Procédé selon la revendication 13, **caractérisé en ce que** la durée d'impulsion ($\tau$) est située dans la plage de 100 $\mu$s à 600 ps.

Fig. 1a

Fig. 1b

Fig. 2

Fig. 3a

Fig. 3d

Fig. 3b

Fig. 3e

Fig. 3c

Fig. 4

Fig. 5a

Fig. 5b

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 192860 A
- WO 213182 A
- WO 234659 A
- WO 219452 A
- EP 2568939T3 A
- JP 2005021263 A
- US 20030050675 A
- WO 200771642 A
- US 2015257970 A1
- US 2018200514 A1
- US 2019091466 A1
- US 2008103462 A1
- US 2005085751 A1